# EUROPEAN PATENT SPECIFICATION

(11) **EP 0 669 344 B1**
(45) Date of publication and mention of the grant of the patent: **08.08.2001**
(21) Application number: 95102569.1
(22) Date of filing: 23.02.1995
(51) Int. Cl.: C07K 16/38, C07K 16/40, C12N 5/20, G01N 33/53

(54) **Monoclonal antibody and immunoassay using the same**
Monoklonaler Antikörper und seine Verwendung in einem Immunoassay
Anticorps monoclonal et son utilisation dans un essai immunologique

(30) Priority: 25.02.1994 JP 2816994
(43) Date of publication of application: 30.08.1995
(60) Divisional application: 00116186.8
(73) Proprietor: DAIICHI PURE CHEMICALS CO. LTD., Chuo-ku Tokyo (JP)
(72) Inventor: Yago, Hirokazu, C/o Daiichi Pure Chemicals, Sumida-ku, Tokyo (JP); Hanada, Hisashi, C/o Daiichi Pure Chemicals, Sumida-ku, Tokyo (JP); Wada, Masato, c/o Daiichi Pure Chemicals, Sumida-ku, Tokyo (JP); Ushizawa, Koji, c/o Daiichi Pure Chemicals, Sumida-ku, Tokyo (JP)
(74) Representative: Kraus, Walter, Dr.

(56) References cited:
- EP-A- 0 391 433
- DATABASE WPI Section Ch, Week 8730 Derwent Publications Ltd., London, GB; Class B04, AN 87-210600 XP002016284 & JP-A-62 138 187 (IATRON LABORATORIES) , 20 June 1987
- BIOCHIMICA ET BIOPHYSICA ACTA , vol. 952, - 1988 pages 37-47, XP000608105 SHINJI ASAKURA ET AL.,: "Preparation and characterization of monoclonal antibodies against the human thrombin-antithrombin III complex"

## Description

The present invention relates to a novel monoclonal antibody and an immunoassay using the same. The immunoassay can accurately detect concentrations of thrombin-antithrombin III complexes in human samples and is useful for diagnosis of a blood clotting tendency.

Human antithrombin III (hereinafter abbreviated to ATIII) is an important inhibitor of serine proteases in the blood clotting system. It inhibits the activity of thrombin as well as the activities of activated factors XII, XI, X and IX. The reaction of ATIII and a serine protease proceeds at a molar ratio of 1:1, wherein the arginine residue of ATIII bonds to the serine residue which is the active center of the serine protease by esterification to form a complex, thereby suppressing the activity of the serine protease. The thrombin-antithrombin III complex (hereinafter abbreviated to TAT) is given as one of such complexes.

An increase in the amount of TAT in human blood is considered to indicate initiation of the blood clotting mechanism and formation of thrombin and other serine proteases in the blood coagulation system by activation thereof. It is therefore considered that a part of behavior in the blood clotting system can be known by measuring the amount of TAT in blood, which may help pathologically determine diseases of patients based on their blood coagulation. For example, it is possible to provide an appropriate therapeutic treatment by early detection of diseases such as thrombogenesis and disseminated intravascular coagulation (DIC).

There are several methods for immunologically measuring TAT in human samples. These include a method of measuring TAT by an inhibition assay of an anti-TAT neoantigen-polyclonal antibody using a TAT labeled with ¹²⁵I (Herbert, L. Lau, The Journal of Biological Chemistry, 255, 5885-5893 (1980)); a method of measuring TAT in human samples by a sandwich system using an antithrombin-polyclonal antibody as a solid phase antibody and anti-ATIII-polyclonal antibody as an enzyme-labeled antibody (H. Pelzer, Thrombosis & Haemostasis, 59, 101-106 (1988)); and the like. A kit using a sandwich system in which a polyclonal antibody conforming to the method of H. Pelzer has been proposed (Japanese Patent Application Laid-open (kokai) 48158/1991) and commercialized.

Because of the use of polyclonal antibodies, all of these methods has a problem associated with homogeneity of the antibodies. That is to say, an unhomogeneous antibody has a high probability of reacting with cross-reactants contained in samples, giving rise to fluctuation in measured values. No methods heretofore proposed could not satisfy the current medical requirements in terms of sensitivity, accuracy, and simplicity.

The use of monoclonal antibodies has been proposed in order to overcome this problem. As anti-TAT monoclonal antibodies, monoclonal antibodies of the neo-antigen of ATIII in the serine protease-ATIII complex which does not react with a solid ATIII or a naturally occurring ATIII are known in the art (S, Asakura et al.. Biochimica Biophysica Acta, 952, 37-47 (1988)); French Patent No. 2645647; and Japanese Patent Application Laid-open (kokai) 138187/1987). It has been impossible, however, to accurately determine the amount of TAT in actual clinical samples such as blood by using these monoclonal antibodies, because TAT is present in these samples in a variety of forms.

The objects of the present invention are therefore to provide monoclonal antibodies which can specifically recognize TAT and exhibit high affinity with TAT, and therefore are useful for the measurement of TAT, and to further provide a method for measuring TAT using these monoclonal antibodies.

Based on the facts that binding of ATIII to a serine protease in the blood clotting system (non-reversible binding) produces a new antigen site (neo-antigen) which does not exist in native ATIII and further that the ATIII changes its structure by binding to heparin (reversible binding), the present inventors assumed that neo-antigens may be produced under different conditions. As a result, the present inventors have found that a neo-antigen similar to the neo-antigen which is produced by binding ATIII to a serine protease can be produced by immobilizing ATIII or by binding an antibody to ATIII. Furthermore, the present inventors have found that prothrombin also produces an epitope which is similar to α-thrombin by immobilization. Based on these findings, the present inventors have been successful in obtaining monoclonal antibodies exhibiting reactivities different from those of conventionally known monoclonal antibodies by introducing, in addition to conventional screening methods, a screening method wherein antibodies exhibiting reactivities for immobilized ATIII and immobilized prothrombin are used, and have found that TAT in clinical samples can be quantitatively analyzed accurately by using these monoclonal antibodies.

An object of the present invention is to provide anti-thrombin-antithrombin III complex monoclonal antibodies which do not react with free prothrombin, free antithrombin III, a combined product of an anti-antithrombin III monoclonal antibody and antithrombin III, immobilized antithrombin III, and antithrombin III reacted with an immobilized serine protease; and react with a free thrombin-antithrombin III complex, an immobilized thrombin-antithrombin III complex, a thrombin-antithrombin III complex reacted with an anti-antithrombin III monoclonal antibody, free thrombin, immobilized thrombin and immobilized prothrombin.

A further object is to provide a method for immunologically measuring a thrombin-antithrombin III complex which comprises bringing a sample to come into contact with two or more monoclonal antibodies selected from the monoclonal antibodies of claim 1 and monoclonal antibodies which do not react with free antithrombin III but react with a neo-antigen of antithrombin III produced for the antithrombin III which reacted with thrombin or a serine protease.

Another object of the present invention is to provide a method for immunologically measuring a thrombin-antithrombin III complex which comprises bringing a sample to come into contact with at least one monoclonal antibody selected from the monoclonal antibodies of claim 1 and at least one monoclonal antibody selected from the monoclonal antibodies which do not react with free antithrombin III; react with both free and immobilized thrombin-antithrombin III complexes; and react with a neo-antigen commonly produced for the antithrombin III reacted with a serine protease, the immobilized antithrombin III and the antithrombin III reacted with an anti-antithrombin III monoclonal antibody and the monoclonal antibodies which do not react with free antithrombin III, but react with a neo-antigen of antithrombin III produced for the antithrombin III reacted with a serine protease.

A further object of the present invention is to provide a process for preparing said monoclonal antibodies and a method for immunologically measuring TAT using the same.

The monoclonal antibodies of the present invention can be prepared from a hybridoma which is obtained by cell fusion of antibody-producing cells of mammals immunized with a TAT and myeloma cells of mammals.

The TAT used as the immunogene is prepared by binding thrombin to ATIII. Any commercially available thrombin and ATIII preparations can be used for the purpose of preparing the TAT. However, because commercially available thrombin preparations contain a large amount of proteins as stabilizers, it is desirable to use α-thrombin obtained by the purification of these commercially available thrombin preparations by anion-exchange resins or affinity chromatography. Thrombin and ATIII are mixed at a molar ratio of 1:1 to 1:5, and the TAT complex formed by binding these is preferably purified by column chromatography.

The thus-obtained TAT is then used as an immunogene for the preparation of hybridoma by a conventional method. Specifically, a TAT is injected to a mammal as the immunogene to immunize it. Although there are no specific limitations to the mammal immunized here, it may be suitably selected taking compatibility with the myeloma cells used in the cell fusion into consideration. Mice and rats are given as specific examples, with BALB/c mice being used commonly. There are no specific limitations to the method by which the mammals are immunized with the immunogene. Any commonly employed immunizing methods can be used. For example, either a TAT or a combination of two or more types of TATs may be injected to mammals by subcutaneous injection, intraperitoneal injection, intravenous injection, intramuscular injection, intrasplenic injection or the like; or orally administered by mixing them with feed or water. As needed, an adjuvant may be used together when the immunization is performed.

Then, splenic cells extracted from the immunized mammal are fused with mouse myeloma cells. As the mouse myeloma cells, those having suitable markers, such as hypoxanthine-guanine phosphoribosyltransferase deficiencies (HGPRT⁻) or thymidine kinase deficiencies (TK⁻), are preferably used. The cell fusion can be carried out according to known methods. Cell fusion accelerators such as polyethylene glycol (PEG) or Sendai virus (HVJ) may be optionally added. The splenic cells and myeloma cells are mixed at a ratio preferably in the range of 1:1 to 10:1. Depending on the situations, the cell fusion may be carried out by the electric fusion method or the like.

The hybridoma can be selectively obtained by culturing the fused cells in a conventional selection medium.

When colonies of the hybridoma has grown sufficiently large, the cell lines producing of the target antibody is retrieved and cloned. The retrieval of the antibody which has a specificity to the antigen can be carried out according to the methods commonly employed for detecting antibodies, for example, the ELISA method, the RIA method or the like.

Monocloning of the selected hybridoma can be carried out, for example, by the limiting dilution method or the soft agar cloning. In this instance, it is desirable to use mouse thymocytes, intraperitoneal macrophages, or other known additives having the equivalent effects as these, as a feeder.

In order to prepare the monoclonal antibody of the present invention using the monocloned hybridoma, the hybridoma is cultured in a suitable medium or in peritoneal cavity of mouse or the like. There are no specific limitations to the medium used here, inasmuch as the same is suitable for the culture of hybridomas. For example, RPMI 1640 medium, containing fetal calf serum, L-glutamine, L-pyruvic acid and antibiotics (penicillin G and streptomycin) is preferably used. The hybridoma is added to the medium at a concentration, for example, of 10⁴-10⁵/ml, and cultured under the conditions of 5% CO₂ at 37°C for 2 to 4 days. The monoclonal antibody of the present invention can be obtained from supernatant which is obtained by centrifuge of the culture broth. The intraperitoneal culture, on the other hand, can be carried out by feeding the hybridoma into peritoneal cavity of a mouse and collecting the abdominal fluid.

The monoclonal antibody of the present invention contained in the supernatant of the culture broth thus obtained can be used as is for the purpose of the present invention. It is more desirable that the supernatant be purified by the fractionation method using ammonium sulfate precipitation, the ion-exchange chromatography, or by using a protein A, an anti-IgG column or the like.

The specificity of the monoclonal antibody thus obtained can be confirmed by the Western Blotting method, the ELISA method using a free antigen, the ELISA method using an immobilized antigen, the ELISA method using an antibody-antigen complex, or the like.

The monoclonal antibodies of the present invention can be grouped into three types according to their reactivity.
(1) The monoclonal antibodies which do not react with free prothrombin, free ATIII, a complex of anti-ATIII monoclonal antibody and ATIII, immobilized ATIII, and ATIII reacted with an immobilized serine protease (the 10th activated blood clotting factor or the like); and react with free TAT, immobilized TAT, TAT reacted with anti-ATIII monoclonal antibody, free thrombin, immobilized thrombin and immobilized prothrombin.
   Monoclonal antibodies No. 26202, 26203 and 26207, which are hereinafter described, are included in this type of monoclonal antibody.
(2) The monoclonal antibodies which do not react with free antithrombin III; react with both free and immobilized TAT; and react with a neo-antigen commonly produced for the ATIII reacted with a serine protease, the immobilized ATIII, and the ATIII reacted with an anti-ATIII monoclonal antibody.
   Monoclonal antibodies No. 26205, 26208, 26209, 26214, 26216, 26218 and 26219, which will be hereinafter described, are included in this type of monoclonal antibody. More specifically, this type of monoclonal antibodies do not react with free ATIII, free thrombin, free prothrombin, immobilized thrombin and immobilized prothrombin; and react with free TAT, immobilized TAT, TAT reacted with anti-ATIII monoclonal antibody, immobilized ATIII, ATIII reacted with an immobilized serine protease, and ATIII reacted with an anti-ATIII monoclonal antibody.

The TAT in samples can be quantitatively analyzed with an excellent accuracy by a conventional immunoassay using one or more monoclonal antibodies of the present invention.

The immunoassay for measuring the TAT content according to the present invention may be carried out by bringing the sample to come into contact with two or more monoclonal antibodies of the present invention. For the accurate determination of TAT without errors due to reactions of the antibodies with TAT, prothrombin or the like, however, a combination of monoclonal antibodies, each exhibiting different reactivities, is preferably used. Specifically, it is preferable to combine two or more monoclonal antibodies selected from aforementioned monoclonal antibodies (1) and (2), and monoclonal antibodies which do not react with free ATIII but react with a neo-antigen of ATIII produced for the ATIII reacted with a serine protease. Among these combinations, the combination of one or more monoclonal antibodies (1) and one or more monoclonal antibodies selected from monoclonal antibodies (2), and the monoclonal antibodies which do not react with free ATIII but react with a neo-antigen of ATIII produced for the ATIII reacted with a serine protease is preferred. Here, included in the monoclonal antibodies which do not react with free ATIII but react with a neo-antigen of ATIII produced for the ATIII reacted with a serine protease are monoclonal antibodies Nos. 26211, 26212, 26217 and 26220, which are described hereinafter. More specifically, these are monoclonal antibodies which do not react with free ATIII, free thrombin, free prothrombin, immobilized ATIII, immobilized thrombin, immobilized prothrombin, and ATIII reacted with an anti-ATIII monoclonal antibody; and which strongly react with free TAT, immobilized TAT, ATIII reacted with an immobilized serine protease, and TAT reacted with an anti-ATIII monoclonal antibody.

There are no specific limitations to the method of immunoassay used in the present invention. For example, the Ouchterlony method, single radial immunodiffusion method, immunonephelometry method, enzyme immunoassay, latex turbidimetric immunoassay, radio immunoassay, fluoro immunoassay, or the like can be employed. Among these methods, when the enzyme immunoassay is used, any one of anti-TAT monoclonal antibody is immobilized to an insoluble carrier in a suitable buffer solution to make it into an insoluble antibody, another anti-TAT monoclonal antibody is labeled with an enzyme, and these monoclonal antibodies are reacted with the sample. The TAT in the sample can then be determined by measuring the activity of the enzyme labeled to the second antibody.

As the insoluble carries used here, various synthetic polymers, such as polystyrene, polyethylene and polypropylene, as well as glasses, silicon, insoluble polysaccharides (crosslinked dextrane, polysaccharides, etc.) and the like are preferred. These carriers may be used in a form of sphere, a rod, fine particles, test tubes or microplates. When the carrier is in the form of sphere, a rod, test tubes or microplates, or it is fine particles, the insolubilized antibody can be prepared using the first and second antibodies at a concentration of 1-10 µg/ml and 1-10 mg/ml, respectively, in a neutral to alkaline (pH 7-10) buffer solution, such as.a phosphate buffer, a glycine buffer, a carbonate buffer or a Tris buffer, at room temperature to 4°C for 1-72 hours.

The enzyme-labeled antibody can be prepared by a known method. For example, it can be prepared, according to the method of Nakane et al. (Nakane P. K et al., J. Histochem Cytochem, 22, 1084-1089 (1974)) or the method of Ishikawa et al. (the maleimide method, "Enzyme immunoassay - third edition", Igaku Shoin), using unfragmented immunoglobulin as is or, as needed, after converting it into F(ab')₂ or Fab' by partial decomposition using a suitable protease. Peroxidase, alkaline phosphatase, β-D-galactosidase, glucose oxidase or the like can be used as the enzyme to be labeled.

When the labeled substance is an enzyme, a substrate for measuring its activity and, as needed, a chromogenic substrate are used. When peroxidase is used as the enzyme, hydrogen peroxide is used as the substrate and o-phenylenediamine, 3,3',5,5'-tetramethylbenzidine, 2,2'-azinodi-[3-ethylbenzthiazoline sulfonic acid]ammonium, or the like is used as the chromogenic substrate; when alkaline phosphatase is used as the enzyme, p-nitrophenyl-phosphate, 3-(4-methoxyspiro{1,2-dioxetane-3,2'-tricyclo-[3.3.1.1^{3,7}]decan}-4-ylphenylphosphate:AMPPD, or the like is used as the substrate; when β-D-galactosidase is used as the enzyme, β-D-galactopyranoside, 4-methyl-umbelliferyl-β-D-galactopyranoside, or the like is used as the substrate; and when glucose oxidase is used as the enzyme, β-D-glucose is used as the substrate in the presence of peroxidase and a chromogenic substrate for peroxidase is used.

The monoclonal antibodies of the present invention specifically recognize TAT and exhibit strong affinity with TAT. Therefore, the quality of TAT in samples can be measured accurately by using this monoclonal antibody without being affected by cross reaction products in the samples. The monoclonal antibody is thus useful for detecting blood clotting tendencies.

Other features of the invention will become apparent in the course of the following description of the exemplary embodiments which are given for illustration of the invention and are not intended to be limiting thereof.

### EXAMPLES

### Example 1

### (1) Preparation of TAT

Because a commercially available thrombin (a product of Green Cross Co., Ltd.) contained a large amount of proteins as a stabilizer, it was purified by the following method. 50,000 units of α-thrombin was dissolved in 50 ml of 50 mM phosphate buffer (pH 7.0, hereinafter referred to simply as phosphate buffer), and the solution was applied to S-Sepharose column (2.5 cm x 11 cm) to linear gradiently elute adsorbed proteins with a phosphate buffer solution containing NaCl (0-0.4 M). Fractions at activity peaks were pooled, while confirming the activity of α-thrombin by the increase in the absorbance at 405 nm using a synthetic substrate, S-2238 (a product of Daiichi Pure Chemical Co., Ltd.). The fractions were then applied to a benzamidine-sepharose 6B column (2 cm x 6 cm) to linear gradiently elute adsorbed proteins with a phosphate buffer solution containing benzamidine (0-0.1 M) and NaCl (0.5 M). After dialyzing against a 20 mM Tris-HCl buffer (pH 7.4) containing 0.1 M NaCl, the purity of the α-thrombin obtained was tested by SDS-PAGE. The α-thrombin was stored at -80°C, at which temperature its autolysis was prevented.

The thus-obtained α-thrombin and a commercially available ATIII preparation (a product of Hoext Co.) was mixed at a molar ratio of 1:1.5 at pH 7.4 and incubated at 37°C for 10 minutes. The resulting reaction product was applied to a heparin-sepharose column (2 cm x 6 cm) because it contained TAT, α-thrombin and TAT degradation products. The adsorbed proteins were eluted with a 20 mM Tris-HCl buffer (pH 7.4) containing 0.1-2 M NaCl to collect TAT fractions after confirmation by SDS-PAGE. The purified TAT thus obtained had a sufficient high purity to be used as the immunogene for the preparation of the monoclonal antibody and its screening. This purified TAT was stored at -80°C to prevent autolysis.

The concentration of the purified TAT was quantitatively analyzed by a protein assay kit (a product of Biolad Co.) which is based on the Bradford colorimetric analysis using BSA as a standard. The concentration was found to be 35 µm/ml and this value was applied to the ELISA and LTIA measurements.

### (2) Immunization

The TAT purified by the above method was used in an amount of 100 mOD at 280 nm for one immunization. Freund's complete adjuvant was used for the first immunization and Freund's incomplete adjuvant was used for the additional immunizations. 100 µl of TAT and 100 µl of Freund's adjuvant were mixed. 200 µl of the resulting emulsion was intraperitoneally injected to a male BALB mouse for one immunization. The immunization was carried out four times at an interval of two weeks.

Besides the Freund's adjuvant, immunization was carried out by using a modified Libie adjuvant system (a product of Funakoshi Co., Ltd.). The immuno-emulsion was prepared by the following method. Using a potter homogenizer, 100 mOD of the purified TAT was added to a grinder tube and immobilized to dryness by injection of nitrogen. A mixture of trehalose dimycolic acid (TDM) and monophosphate lipid A (MPL) dissolved in a 4:1 chloroform-methanol mixed solvent was further added, followed by nitrogen injection for solidification to dryness. After the addition of 4 µl of squalene to this tube, the antigen, adjuvant and squalene were stirred for 3 minutes at 1200 rpm using a teflon rod set in a three-one motor. Then, 200 µl of a 13 mM phosphate buffer (pH 7.2) containing 0.2% Tween 80 and 0.72% NaCl was added and the mixture was stirred for 4 minutes at 1200 rpm, thus obtaining the immuno-emulsion. 200 µl of the emulsion was intraperitoneally injected to a male BALB mouse for one immunization. The immunization was carried out four times at an interval of two weeks. For the first immunization 50 µg of TDM and 50 µg of MPL were used, and for the additional immunizations 50 µg of TDM and 5 µg of MPL were used.

Blood was collected from ophthalmo veins of the mice immunized by these two methods to measure the antibody titer by the ELISA method. The mouse having a higher value of antibody titer was selected for the cell fusion.

### (3) Cell fusion

Four weeks after the fourth immunization, 100 mOD of the purified TAT diluted with 200 µl of a physiological saline was intraperitoneally injected to the mouse. Three days thereafter, the spleen was extracted, well loosened with tweezers and ground slide glass in a medium (PRMI 1640), and splenic cells were collected by centrifuge at 1500 rpm for 5 minutes. The splenic cells thus collected were washed with the same medium and again centrifuged. Finally, 2 ml of the same medium containing 15% FCS was added to the cells to obtain a splenic cell suspension. The number of living splenic cells, in a 1:1 mixture of this suspension and a solution of acridine orange and ethydium bromide (0.1 mg of each was dissolved in 1 ml of PBS), were counted by a fluorescent microscope. The living splenic cells (10⁸) were mixed with myeloma cells (10⁷) of a SP2/O·Ag14 mouse, which were cultured in advance and at the stage of logarithmic growth, and the mixture was centrifuged at 1500 rpm for 5 minutes. After removing the supernatant, the cells were well loosened, suspended in a GKN solution (containing 8 g of NaCl, 0.4 g of KCl, 2 g of glucose, 1.41 g of Na₂HPO₄ and 0.78 g of NaH₂PO₄·H₂O in 1 1 of purified water), centrifuged at 1500 rpm for 5 minutes. After repeating this washing procedure, 0.5 ml of a GKN solution containing 50% (w/v) polyethylene glycol 1540 was slowly added and the mixture was gently stirred for one minute. To the mixture was slowly and gently added 10 ml of the GKN solution to terminate the reaction, followed by centrifuge at 1500 rpm for 5 minutes. The cells thus obtained were suspended in 30 ml of RPMI 1640 solution containing 15% FCS, charged to 3 sheets of 96-well microculture plates, 0.1 ml per well, which contained HAT medium (RPMI 1640 medium containing 10⁻⁴ hypoxanthine, 4x10⁻⁷ aminopterin, 1.5x10⁻⁵ thymidine, and 15% FCS) and feeder cells (200 µl per well), and cultured in a 5% CO₂ incubator at 37°C. After 10 days, growth of fused cells was confirmed in all wells.

### (4) Selection and cloning of anti-TAT antibody-producing hybridoma

The presence or absence of anti-TAT antibody in the supernatant of the culture broth was measured by the ELISA method, wherein 6 sheets of microplates: a 96-well microplate with immobilized purified TAT (0.5 mOD), a 96-well microplate with immobilized ATIII (2 µg/ml), a 96-well microplate with immobilized α-thrombin (2 µg/ml), a 96-well microplate with immobilized prothrombin (2 µg/ml), a 96-well microplates with immobilized anti-ATIII monoclonal antibody (F(ab')₂, 5 mOD), and a 96-well microplates wherein TAT (0.5 mOD) and ATIII (2 µg/ml) were bound, were used for performing the screening.

Specifically, each concentration of TAT or monoclonal antibody was diluted with a 13 mM phosphate buffer (pH 7.2, PBS) containing 0.72% NaCl, charged to 96-well microplates in an amount of 50 µl/well, allowed to stand overnight at 4°C, and washed three times with PBS containing 1% bovine serum albumin and 0.05% Tween 20 (pH 7.2, BSA-PBS). Further, regarding the 96-well microplates wherein each antigen was bound, the each antigen was diluted with BSA-PBS, charged to the 96-well microplates in an amount of 50 µl/well, and allowed to stand overnight at 4°C, thereby preparing plates for the screening. The supernatant of the culture broth was charged to each plate at a proportion of 50 µl/well, incubated for one hour at 37°C, and washed three times with PBS. Then, 50 µl of a peroxidase-labeled anti-mouse IgG antibody (having specificity to the Fc position, originated from goat), diluted to a 1000-fold with BSA-PBS, was added, followed by incubation at 37°C for one hour and then washing with PBS five times. After the addition of a citrate-phosphate buffer (pH 5.00) containing 0.2% o-phenylenediamine and 0.02% hydrogen peroxide aqueous solution at a proportion of 50 µl/well, the reaction was carried out at room temperature for 30 minutes and terminated by the addition of a 4.5 M sulfuric acid aqueous solution at a proportion of 50 µl/well. Wells producing supernatant with a high absorbance at 550 nm as the result of this reaction were selected.

The immobilized monoclonal antibody was converted into F(ab')2 by the pepsin treatment so that it does not react with the peroxidase-labeled anti-mouse IgG antibody (having specificity to Fc position, originated from goat) used in the ELISA method, and purified by DEAE-Sephacel (an cationic exchange resin) in order to remove unreacted IgG fractions.

Monocloning was carried out by the limiting dilution method by charging 0.1 ml/well of diluted hybridomas (10 per ml) exhibiting a positive reaction to the specific antibody to a 96-well microculture plate, to which 10⁶/0.2 ml per well of BALB/c mouse thymocytes were added as feeder cells. As the culture media, HAT medium for the first culture, HT medium for the second culture, and PPMI 1640 containing 15% FCS for the third culture and thereafter, were used. The cells were cultured for 10 days in a 5% CO₂ incubator at 37°C.

The selection of specific antibody positive wells by the ELISA method and the monocloning by the limiting dilution method were repeated three times. The screenings, the second time and thereafter, were performed only on the TAT immobilized plates. As a result, 17 strains of hybridomas capable of producing anti-TAT monoclonal antibody were obtained.

These 17 strains of hybridomas were named 26202, 26203, 26205, 26207, 26208, 26209, 26211, 26212, 26214, 26216, 26217, 26218, 26219 and 26220. Among these, 7 strains were deposited with National Institute of Bioscience and Human-Tachnology, Agency of Industrial Science and Technology, under the deposition numbers shown in Table 1.

**TABLE 1**

| Hybridoma | Deposition No. |
|---|---|
| 26203 | FERM BP-5003 |
| | |
| 26207 | FERM P-14096 |
| 26208 | FERM P-14097 |
| 26209 | FERM BP-5004 |
| | |
| 26212 | FERM P-14100 |

The class and sub-class for each monoclonal antibody were determined using the Mono Ab-ID EIA kit. The results are shown in Table 2.

**TABLE 2**

| Classes and sub-classes of anti-TAT monoclonal antibodies | | | |
|---|---|---|---|
| Monoclonal antibody | Class | Sub-class | L-chain |
| 26202 | G | 1 | κ |
| 03 | G | 1 | κ |
| 05 | G | 2a | κ |
| | | | |
| 07 | G | 1 | κ |
| 08 | G | 2a | κ |
| 09 | G | 2b | κ |
| | | | |
| 11 | G | 1 | κ |
| 12 | G | 1 | κ |
| 13 | G | 2b | κ |
| 14 | G | 1 | κ |
| 16 | G | 1 | κ |
| 17 | G | 1 | κ |
| 18 | G | 1 | κ |
| 19 | G | 1 | κ |
| 20 | G | 1 | κ |

### (5) Separation and purification of monoclonal antibody

The hybridomas capable of producing the anti-TAT monoclonal antibodies obtained in (4) above were cultured in mouse peritoneal to produce monoclonal antibodies.

As a pretreatment, 0.5 ml of bristane (2,6,10,14-tetramethylpentadecane) was intraperitoneally injected into a BALB/c mouse, aged 8 weeks. After 8 days, 4-15 x 10⁵ hybridomas suspended in 0.5 ml of RPMI 1640 medium were intraperitoneally injected into the mouse. Starting from 9 days after the injection, abdominal dropsy was repeatedly taken out and pooled. The collected abdominal dropsy was centrifuged at 3000 rpm for 10 minutes to remove insoluble matters such as cells. An equivalent volume of saturated ammonium sulfate solution was added to the supernatant while stirring, following which the mixture was allowed to stand overnight at 4°C. The precipitate produced was collected by centrifuge, dissolved in a 20 mM Tris-HCl buffer (pH 8.0), and dialyzed. The dialyzed product was adsorbed in a DEAE-Sephacel column which was equilibrated with the same buffer solution and eluted linear gradiently with 0-0.3 M NaCl in the same buffer, thus obtaining purified antibodies.

### Example 2

### <Reaction specificity of the monoclonal antibodies>

### (1) Western Blotting

The reaction specificities of the 17 monoclonal antibodies obtained above were confirmed by the Western Blotting method. In the SDS-PAGE using 4-20% SDS, a reactant of α-thrombin (1 µg/well) and ATIII (2.6 µg/well) at 37°C for 5 minutes, added with α-thrombin (1 µg/well), was applied. Further, Russell's viper venom (RVV, 20 ng) was added to FX (1 µg) and reacted at 37°C for 5 minutes. To the product was added ATIII (2 µg) and the mixture was further reacted at 37°C for 5 minutes. FX and FXa (0.5 µg each) were added to the resulting product, and this mixture was also applied. After electrophoresis, these samples were transcripted onto a nitrocellulose membrane using a transcription buffer consisting of 25 mM Tris buffer, 192 mM glycine, and 20% methanol at 40 V/5 cm for 4 hours. The nitrocellulose membrane was blocked using BSA-PBS at 4°C overnight before it is reacted with each monoclonal antibody obtained in this experiment. The nitrocellulose membrane was cut into strips. 500 µl of the culture broth supernatant of each hybridoma was reacted on each strip at room temperature for one hour. The strips were washed three times with PBS containing 0.05% Tween 20 (PBST) and reacted with 500 µl of peroxidase-labeled anti-mouse IgG antibody (having specificity to Fc position) which was diluted to a 1000-fold with BSA-PBS, at room temperature for one hour. Further, after washing three times with PBST, a substrate solution containing 100 ml of a 50 mM Tris-HCl buffer (pH 7.6), 25 mg of diaminobenzidine, and 20 µl of hydrogen peroxide was added to carry out an enzymatic reaction. The reaction was terminated by washing with water immediately after bands were confirmed. The results are shown in Table 3.

The 17 monoclonal antibodies reacted with TAT, α-thrombin and FXa·AT, but did not react with anything other than these. Based on the results, the 20 monoclonal antibodies were confirmed to be the antibodies against neo-antigens of α-thrombin and ATIII.

**TABLE 3**

| Reactivities of anti-TAT monoclonal antibodies in Western Blotting | | | | | | |
|---|---|---|---|---|---|---|
| Antibody | TAT | ATIII | T | FXa•AT | FXa | FX |
| 26202 | + | - | + | - | - | - |
| 03 | + | - | + | - | - | - |
| 05 | + | - | - | + | - | - |
| | | | | | | |
| 07 | + | - | + | - | - | - |
| 08 | + | - | - | + | - | - |
| 09 | + | - | - | + | - | - |
| | | | | | | |
| 11 | + | - | - | + | - | - |
| 12 | + | - | - | + | - | - |
| | | | | | | |
| 14 | + | - | - | + | - | - |
| 16 | + | - | - | + | - | - |
| 17 | + | - | - | + | - | - |
| 18 | + | - | - | + | - | - |
| 19 | + | - | - | + | - | - |
| 20 | + | - | - | + | - | - |

| | | | | | | |
|---|---|---|---|---|---|---|
| TAT: Thrombin-antithrombin III complex | | | | | | |
| ATIII: Antithrombin III | | | | | | |
| T: Thrombin | | | | | | |
| FXa: Activated blood clotting factor 10 | | | | | | |
| FXa·AT: FXa-antithrombin III complex | | | | | | |
| +, -: Results obtained by naked eye observation | | | | | | |

As shown in Table 3, antibodies No. 26202, 26203 and 26207 reacted with TAT and α-thrombin, but not with other antigens, revealing that these are antibodies for TAT and α-thrombin. Other antibodies reacted with TAT and FXa·AT, but not with other antigens. These were thus confirmed to be the antibodies for the antibodies of neo-antigens of TAT and ATIII.

### (2) ELISA (Immobilized antigens)

The reaction specificities of the above 17 monoclonal antibodies were confirmed by the ELISA method. TAT (0.5 mOD), ATIII (2 µg/ml), α-thrombin (2 µg/ml), prothrombin (2 µg/ml), FXa (0.5 µgm/l as FX, a reaction product of 10 µg of FX and 0.2 µg of RVV at 37°C for 15 minutes), and FXa·AT (0.5 µg/ml as FX, a reaction product of 5.5 µg of FXa and 5 µg of ATIII at 37°C for 5 minutes, which contains almost no free ATIII) were diluted with PBS and charged to 96-well microplates in an amount of 50 µl/well. The plates were allowed to stand overnight at 4°C and washed three times with BSA-PBS. These plates were served for the test of reaction specificities with immobilized antigens. The procedure for the selection of hybridomas in Example 1(4) was applied to the reaction of the monoclonal antibodies and thereafter in this test. The results are shown in Table 4.

**TABLE 4**

| Reactivities of anti-TAT monoclonal antibodies and immobilized antigens by the ELISA method | | | | | | |
|---|---|---|---|---|---|---|
| | Antigens used for sensitizing the plates | | | | | |
| Antibody | TAT | ATIII | T | ProT | FXa | FXa·AT |
| 26202 | +++ | - | +++ | +++ | - | - |
| 03 | +++ | - | +++ | +++ | - | - |
| 05 | +++ | +++ | - | - | - | +++ |
| | | | | | | |
| 07 | +++ | - | +++ | +++ | - | - |
| 08 | +++ | + | - | - | - | ++ |
| 09 | +++ | + | - | - | - | +++ |
| | | | | | | |
| 11 | +++ | - | - | - | - | + |
| 12 | +++ | - | - | - | - | ++ |
| | | | | | | |
| 14 | +++ | + | - | - | - | + |
| 16 | +++ | ++ | - | - | - | + |
| 17 | +++ | - | - | - | - | + |
| 18 | +++ | ++ | - | - | - | ++ |
| 19 | +++ | ++ | - | - | - | ++ |
| 20 | +++ | - | - | - | - | ++ |

| | | | | | | |
|---|---|---|---|---|---|---|
| TAT: Thrombin-antithrombin III complex | | | | | | |
| ATIII: Antithrombin III | | | | | | |
| T: Thrombin | | | | | | |
| ProT: Prothrombin | | | | | | |
| FXa: Activated blood clotting factor 10 | | | | | | |
| FXa·AT: FXa-antithrombin III complex +, -: Absorbance at 550 nm. The following criteria were applied to the results: | | | | | | |
| +++: ≥ 500 mOD | | | | | | |
| ++: 500 > - ≥ 300 mOD | | | | | | |
| +: 300 > - ≥ 100 mOD | | | | | | |
| -: 100 mOD > | | | | | | |

### (3) ELISA (antibody-bound antigens)

Two types of anti-ATIII monoclonal antibodies (5 mOD each), both converted into F(ab'), were diluted with PBS and charged to 96-well microplates in an amount of 50 µl/well. The plates were allowed to stand overnight at 4°C and washed three times with BSA-PBS. TAT (0.5 mOD) and ATIII (2 µg/ml) were diluted with PBS and charged to 96-well microplates at a proportion of 50 µl/well. The plates were allowed to stand overnight at 4°C to prepare antibody-bound antigen plates. These plates were served for the test of reaction specificities with antibody-bound antigens. The procedure for the selection of hybridomas in Example 1(4) was applied to the reaction of the monoclonal antibodies and thereafter in this test. The results are shown in Table 5.

**TABLE 5**

| Reactivities of anti-TAT monoclonal antibodies and antibody-bound antigens by the ELISA method | | | | |
|---|---|---|---|---|
| Antibody | 13206 | | 13208 | |
| | TAT | ATIII | TAT | ATIII |
| 26202 | +++ | - | +++ | - |
| 03 | +++ | - | +++ | - |
| 05 | +++ | +++ | +++ | + |
| | | | | |
| 07 | +++ | - | +++ | - |
| 08 | +++ | +++ | +++ | + |
| 09 | +++ | +++ | +++ | + |
| | | | | |
| 11 | +++ | - | +++ | - |
| 12 | +++ | - | +++ | - |
| | | | | |
| 14 | +++ | +++ | +++ | - |
| 16 | +++ | - | +++ | + |
| 17 | +++ | - | +++ | - |
| 18 | +++ | +++ | +++ | + |
| 19 | +++ | +++ | +++ | ++ |
| 20 | +++ | - | +++ | - |

| | | | | |
|---|---|---|---|---|
| TAT: Thrombin-antithrombin III complex | | | | |
| ATIII: Antithrombin III | | | | |
| 13206, 13208: Anti-ATIII monoclonal antibodies with different recognizing epitope +, -: Absorbance at 550 nm. The following criteria were applied to the results: | | | | |
| +++: ≧ 500 mOD | | | | |
| ++: 500 > - ≧ 300 mOD | | | | |
| +: 300 > - ≧ 100 mOD | | | | |
| -: 100 mOD > | | | | |

### (4) ELISA (free antigens)

TAT (0.1 mOD) was diluted with PBS and charged to a 96-well microplate in an amount of 50 µl/well. The plate was allowed to stand overnight at 4°C and washed three times with BSA-PBS. TAT (100 mOD), ATIII (100 µg/ml), α-thrombin (100 µg/ml) or prothrombin (100 µg/ml) was diluted with PBS and charged to 96-well microplates in an amount of 50 µl/well. Further, the supernatant of each monoclonal antibody culture broth was charged to the 96-well microplates in an amount of 50 µl/well, and reacted at 37°C for one hour. The procedure for the selection of hybridomas in Example 1(4) was applied to the reaction with peroxidase-labeled antibody and thereafter in this test. The results are shown in Table 6.

**TABLE 6**

| Reactivities of anti-TAT monoclonal antibodies and free antigens by the ELISA method | | | | |
|---|---|---|---|---|
| Antibody | Free antigens | | | |
| | TAT | ATIII | T | ProT |
| 26202 | ++ | - | ++ | - |
| 03 | ++ | - | ++ | - |
| 05 | ++ | - | - | - |
| | | | | |
| 07 | ++ | - | + | - |
| 08 | + | - | - | - |
| 09 | + | - | - | - |
| | | | | |
| 11 | + | - | - | - |
| 12 | + | - | - | - |
| | | | | |
| 14 | + | - | - | - |
| 16 | ++ | - | - | - |
| 17 | + | - | - | - |
| 18 | + | - | - | - |
| 19 | + | - | - | - |
| 20 | + | - | - | - |

| | | | | |
|---|---|---|---|---|
| TAT: Thrombin-antithrombin III complex | | | | |
| ATIII: Antithrombin III | | | | |
| T: Thrombin | | | | |
| ProT: Prothrombin +. -: Each anti-TAT monoclonal antibody was reacted in the presence of immobilized TAT and each free antigen to measure the absorbance. Taking the absorbance of a sample with no free antigens as 100%, the rates of inhibition by free antigens were shown according to the following criteria: +++: 30% > - ≥ 0% | | | | |
| ++: 50% > - ≥ 30% | | | | |
| +: 70% > - ≥ 50% | | | | |
| -: ≥ 70% | | | | |

From Tables 4, 5 and 6 the 17 monoclonal antibodies of the present invention are found to be grouped into the aforementioned groups (1), (2) and (3).

### Example 3

### <Enzyme immunoassay>

### (1) preparation of peroxidase-labeled antibodies

Horse radish peroxidase (HRP) was used as the enzyme for labeling antibodies. The labeling was performed according to the method of Nakane et al. (Nakane et al., J. Histochem, 22, 1084-1089 (1974)). Unfragmented 17 IgG molecules, each in an amount equivalent to 5 OD, were dialyzed in a 0.2 M carbonate buffer (pH 9.5) to remove Tris and concentrated to a volume of about 500 µl using collodion bag. 5 mg HRP was used for each antibody. 5 mg of HRP (a product of Toyobo Co., Ltd.) was dissolved in 1 ml of distilled water and 75 µl of 0.1 M NaIO₄ was added to the solution, followed by stirring for 20 minutes at room temperature. The resulting solution was dialyzed against a 1 mM acetate buffer (pH 4.0) to decrease the pH to around 4, following which 100 µl of a 0.2 M carbonate buffer (pH 9.5) was added to increase the pH to about 9. The solution was mixed with the above IgG solution and stirred at room temperature for 2 hours for effecting labeling of IgG and HRP. The reaction was terminated by the addition of 100 µl of a 4 mg/ml NaBH₄ solution. After dialyzing with PBS, the product was stored at 4°C.

### (2) Measurement of TAT

The antibody (IgG) was diluted with PBS to a concentration of 4 mOD and charged to 96-well microplates in an amount of 100 µl/well and allowed to stand overnight at 4°C to fix the antibody. PBS was removed by aspiration, 300 µl/well of BSA-PBS was added, and the plates were left at room temperature for one hour. After removing BSA-PBS by aspiration, diluents of TAT (3 ng/ml-60 ng/ml, six diluents) with BSA-PBS were added, each in an amount of 100 µl/well. After the reaction for 2 hours, the products were washed three times with PBS. The HRP-labeled monoclonal antibodies were diluted with BSA-PBS to a concentration of 8 mOD, added to the 96-microplates in an amount of 100 µl/well, reacted for 2 hours at room temperature, and washed five times with PBS. Then, an enzymatic reaction was carried out using H₂O₂ as a substrate and o-phenylenediamine as a chromogenic substrate, and activities were measured by absorbances at 492 nm and 690 nm. The substrate solutions were prepared according to the same procedure as applied to the selection of hybridomas in Example 1(4). Absorbances measured on TAT (10 ng/ml), ATIII (300 µg/ml) and prothrombin (170 µg/ml) in the enzyme immunoassay are shown in Table 7, wherein the absorbances from which a blank value at 492/690 have been subtracted are given.

**TABLE 7**

| Reactivities of antigens in the enzyme immunoassay | | | |
|---|---|---|---|
| Solid phase/label combination | TAT 10 ng/ml | ATIII 300 µg/ml | Prothrombin 170 µg/ml |
| 26203/08 | 1.400 | 0.005 | 0.000 |
| 26203/09 | 2.076 | 0.000 | 0.000 |
| | | | |
| 26207/08 | 1.450 | 0.074 | 0.000 |
| 26207/09 | 1.714 | 0.016 | 0.032 |
| 26212/09 | 2.177 | 0.016 | 0.000 |

### Example 4

### <Latex turbidimetric immunoassay>

### (1) Preparation of latexes sensitized with monoclonal antibodies

Solutions of each antibody at a concentration of 1 OD (absorbance at 280 nm) prepared by diluting the antibody with a 50 mM glycine buffer (pH 9.6) containing 5 mM EDTA·2Na and 150 mM NaCl were mixed with a suspension of latex (φ 0.12 µm), which was diluted with the same buffer solution to a concentration of 1%, at a volume ratio of 1:1. After homogenizing 3 hours at 4°C using a wave roter, the mixture was centrifuged at 25000 x g for one hour to collect latex. The supernatant was removed by aspiration, and to the precipitate was added a twice volume of the same buffer solution containing 0.1% BSA and 0.005% Tween 80. After homogenizing about 17 hours at 4°C using a wave roter, the mixture was centrifuged at 25000 x g for one hour to collect latex. The supernatant was removed by aspiration, and to the precipitate was added a twice volume of the same buffer solution containing 0.1% BSA and 10% glycerol. The mixture was homogenized overnight at 4°C using a wave roter to prepare a suspension. This suspension was used as the antibody sensitized latex for the measurement.

### (2) Measurement of TAT

300 µl of a 100 mM phosphate buffer (pH 7.2), containing 1% BSA, 5 mM EDTA•2Na and 150 mM NaCl, and two latex suspensions sensitized with antibody prepared above, 50 µl each, were mixed. This mixture was mixed with 100 µl of a TAT solution diluted to a prescribed concentration with a 100 mM phosphate buffer (pH 7.2) containing 1% BSA. After 5 minutes, the absorbance at 600 nm was measured on the resulting mixture. The same phosphate buffer solution, not containing TAT, was used instead of samples as the blank. Absorbances measured on TAT (1 µg/ml), ATIII (300 µg/ml) and prothrombin (170 µg/ml) in this latex turbidimetric immunoassay are shown in Table 8, wherein absorbances at 600 nm at five minutes after the reaction are shown.

**TABLE 8**

| Reactivities of antigens in the latex immunoturbidimetric immunoassay | | | |
|---|---|---|---|
| Solid phase/label combination | TAT 1 µg/ml | ATIII 300 µg/ml | Prothrombin 170 µg/ml |
| 26203/10 | 1.149 | -0.034 | -0.024 |
| | | | |
| Mouse IgG | -0.023 | ND | ND |
| ND: Not done. | | | |

## Claims

1. Anti-thrombin-antithrombin III complex monoclonal antibodies which do not react with free prothrombin, free antithrombin III, a combined product of an anti-antithrombin III monoclonal antibody and antithrombin III, immobilized antithrombin III, and antithrombin III reacted with an immobilized serine protease; and react with a free thrombin-antithrombin III complex, an immobilized thrombin-antithrombin III complex, a thrombin-antithrombin III complex reacted with an anti-antithrombin III monoclonal antibody, free thrombin, immobilized thrombin and immobilized prothrombin.

2. A method for immunologically measuring a thrombin-antithrombin III complex which comprises bringing a sample to come into contact with two or more monoclonal antibodies selected from the monoclonal antibodies of claim 1 and monoclonal antibodies which do not react with free antithrombin III but react with a neo-antigen of antithrombin III produced for the antithrombin III which reacted with thrombin or a serine protease.

3. A method for immunologically measuring a thrombin-antithrombin III complex which comprises bringing a sample to come into contact with at least one monoclonal antibody selected from the monoclonal antibodies of claim 1 and at least one monoclonal antibody selected from the monoclonal antibodies
which do not react with free antithrombin III; react with both free and immobilized thrombin-antithrombin III complexes; and react with a neo-antigen commonly produced for the antithrombin III reacted with a serine protease, the immobilized antithrombin III and the antithrombin III reacted with an anti-antithrombin III monoclonal antibody
and the monoclonal antibodies which do not react with free antithrombin III, but react with a neo-antigen of antithrombin III produced for the antithrombin III reacted with a serine protease.

## Patentansprüche

1. Monoclonale Anti-Thrombin-Antithrombin-III-Komplex-Antikörper, die nicht mit freiem Prothrombin, freiem Antithrombin III, einem kombinierten Produkt eines Anti-Antithrombin-III monoclonalen Antikörpers und Antithrombin III, immobilisiertem Antithrombin III und Antithrombin III, umgesetzt mit einer immobilisierten Serinprotease, reagieren; und die mit einem freien Thrombin-Antithrombin-III-Komplex, immobilisiertem Thrombin-Antithrombin-III-Komplex, Thrombin-Antithrombin-III-Komplex, umgesetzt mit einem monoclonalen Anti-Antithrombin-III-Antikörper, freiem Thrombin, immobilisiertem Thrombin und immobilisiertem Prothrombin reagieren.

2. Verfahren zur immunologischen Messung eines Thrombin-Antithrombin-III-Komplexes, umfassend das Inkontaktbringen einer Probe mit zwei oder mehreren monoclonalen Antikörpern, ausgewählt aus den monoclonalen Antikörpern gemäß Anspruch 1 und monoclonalen Antikörpern, die nicht mit freiem Antithrombin III reagieren, jedoch mit einem Ne'o-Antigen von Antithrombin III, das für Antithrombin III, das mit Thrombin oder einer Serinprotease reagierte, hergestellt wurde, reagieren.

3. Verfahren zur immunologischen Messung eines Thrombin-Antithrombin-III-Komplexes, umfassend das Inkontaktbringen einer Probe mit wenigstens einem monoclonalen Antikörper, ausgewählt aus dem monoclonalen Antikörper nach Anspruch 1, und wenigstens einem monoclonalen Antikörper, ausgewählt aus den monoclonalen Antikörpern, die nicht mit freiem Antithrombin III reagieren; sowohl mit freien als immobilisierten Thrombin-Antithrombin-III-Komplexen reagieren und mit einem Neo-Antigen reagieren, das in üblicher Weise für das Antithrombin III, das mit einer Serinprotease reagierte, hergestellt wurde, wobei das immobilisierte Antithrombin III und das Antithrombin III mit einem monoclonalen Anti-Antithrombin-III-Antikörper und den monoclonalen Antikörpern, die nicht mit freiem Antithrombin III reagieren, die jedoch mit einem Neo-Antigen des Antithrombin III, hergestellt aus dem Antithrombin III, umgesetzt mit einer Serinprotease, reagieren.

## Revendications

1. Anticorps monoclonaux anti-complexe thrombine-antithrombine III qui ne réagissent pas avec la prothrombine libre, avec la prothrombine III libre, avec un produit combiné d'un anticorps monoclonal anti-antithrombine III et d'antithrombine III, avec l'antithrombine III immobilisée et avec l'antithrombine III ayant réagi avec une sérine-protéase immobilisée ; et qui réagissent avec un complexe thrombine-antithrombine III libre, avec un complexe thrombine-antithrombine III immobilisé, avec un complexe thrombine-antithrombine III ayant réagi avec un anticorps monoclonal anti-antithrombine III, avec la thrombine libre, avec la thrombine immobilisée et avec la prothrombine immobilisée.

2. Procédure de mesure par voie immunologique d'un complexe thrombine-antithrombine III qui comprend la mise en contact d'un échantillon avec deux ou plusieurs anticorps monoclonaux choisis parmi les anticorps monoclonaux de la revendication 1 et les anticorps monoclonaux qui ne réagissent pas avec l'antithrombine III libre mais réagissent avec un néo-antigène de l'antithrombine III produit pour l'antithrombine III ayant réagi avec la thrombine ou une sérine-protéase.

3. Procédure de mesure par voie immunologique d'un complexe thrombine-antithrombine III qui comprend la mise en contact d'un échantillon avec au moins un anticorps monoclonal choisi parmi les anticorps monoclonaux de la revendication 1, et avec au moins un anticorps monoclonal choisi parmi les anticorps monoclonaux qui ne réagissent pas avec l'antithrombine III libre; qui réagissent avec les complexes thrombine-antithrombine III tant libres qu'immobilisés ; et qui réagissent avec un néo-antigène communément produit pour l'antithrombine ayant réagi avec une sérine-protéase, avec l'antithrombine III immobilisée et avec l'antithrombine III ayant réagi avec un anticorps monoclonal anti-antithrombine III, et les anticorps monoclonaux qui ne réagissent pas avec l'antithrombine III mais réagissent avec un néo-antigène de l'antithrombine III produit pour l'antithrombine III ayant réagi avec la thrombine ou une sérine-protéase.
